Europäisches Patentamt

European Patent Office

Office européen des brevets

(11) Veröffentlichungsnummer: **0 362 732**
**A2**

## (12) EUROPÄISCHE PATENTANMELDUNG

(21) Anmeldenummer: 89118145.5

(51) Int. Cl.⁵: **C12N 15/86**

(22) Anmeldetag: 30.09.89

Patentansprüche für folgende Vertragsstaaten:
ES + GR.

(30) Priorität: 07.10.88 DE 3834157

(43) Veröffentlichungstag der Anmeldung:
**11.04.90 Patentblatt 90/15**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Fleckenstein, Bernhard, Prof. Dr.**
**Schlaifhausen 93**
**D-8551 Wiesenthau(DE)**
Erfinder: **Grassmann, Ralph**
**Rennesstrasse 26**
**D-8520 Erlangen(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) Selektierbare Vektoren für humane T-Zellen.

(57) Selektierbare Herpesvirus saimiri Vektoren werden beschrieben, die ein Selektionsgen in einer Verbindungsregion der L- und H-DNA insertiert haben. Solche Vektoren sind in der Lage, humane T-Zellen persistent zu infizieren und damit zur Expression von Fremdgenen in humanen T-Zellen geeignet. Ein zusätzlicher Vorteil besteht darin, daß dabei keine infektiösen Viruspartikel produziert werden.

EP 0 362 732 A2

## Selektierbare Vektoren für humane T-Zellen

Die Erfindung betrifft selektierbare Vektoren, die die Expression von Fremdgenen in humanen T-Zellen ermöglichen.

Viren der Herpesgruppe (Herpes Simplex Virus, Cytomegalovirus, Varicella/Zoster Virus oder Herpesvirus suis) konnten erfolgreich eingesetzt werden, um heterologe Gene in lytisch infizierten "Monolayer" Zellkulturen zu exprimieren. Es gibt jedoch keine Zellinien, die durch diese Viren für längere Zeit persistent infiziert sind. Persistierende Vektoren wurden aus dem Genom von Epstein-Barr Virus (EBV) konstruiert, wobei aber aus den infizierten B-Lymphozyten keine infektiösen, zellfreien Viren erhalten werden konnten.

Es war ebenfalls bekannt, daß Herpesvirus (H.) saimiri sowohl sich lytisch in epitheloiden Zellen vermehrt als auch T-Zellen von Krallenaffen persistent infiziert. Das Genom von H. saimiri besteht aus einer Region von etwa 112 kb einer "unique" DNA niederen GC-Gehalts (L-DNA). Sie ist an beiden Enden von nicht-kodierenden tandemartig angeordneten "Repeat"-Einheiten variabler Zahl (H-DNA) flankiert, die eine Länge von je etwa 1.4 kb besitzen und GC-reich sind. H. saimiri induziert schnell wachsende T-Zell-Lymphome in einigen Primaten der Neuen Welt und kann Krallenaffen-T-Lymphozyten in vitro zu permanentem Wachstum transformieren.

Wir haben gefunden, daß ein rekombinantes H. saimiri Virus, das das neo-Gen enthält, in hoher Zahl durch Geneticin (G418) in Affennierenzellinien selektiert wird. Die Bedingungen für homologe Rekombination waren so gewählt, daß das neo-Gen, das hier als Beispiel für ein beliebiges selektierbares Gen steht, in die rechte Verbindungsstelle der H- und L-DNA insertiert ist. Diese Rekombinanten sind in der Lage, humane T-Zellinien persistent zu infizieren, wobei die virale DNA als Episom vorliegt. Da das virale Genom etwa 30% hoch-repetitive DNA enthält, die durch heterologe DNA ersetzbar ist, stehen damit Vektoren zur Expression von Fremdgenen in humanen T-Zellen zur Verfügung.

Ein Vorteil solcher Expressionssysteme ist ferner darin zu sehen, daß persistent infizierte Zellen als "non-producer cells" keine infektiösen Viruspartikel produzieren. Als Ausgangsstämme können auch replikationskompetente, nicht onkogene Varianten von H. saimiri eingesetzt werden. Diese Virusstämme weisen Deletionen in der linksterminalen L-DNA auf (B. Fleckenstein u. R. Desrosiers (1982) , "The Herpes viruses, Vol. 1, B. Roizman (ed.). Plenum Publishing Co., New York).

Bisher wurde nicht gezeigt, daß H. saimiri humane lymphatische Zellen infiziert.

Die Erfindung betrifft folglich:

a) Selektierbare H. saimiri Rekombinanten, die ein Selektionsgen in der rechten oder linken Verbindungsstelle bzw. Verbindungsregion der H- und L-DNA insertiert haben,

b) Verfahren zur Herstellung der unter a) genannten Rekombinanten und

c) ihre Verwendung zur Expression von Fremdgenen in humanen T-Lymphozyten, sowie

d) die Erzeugung von Affen T-Zellinien, die ein Fremdgen konstitutiv exprimieren, mittels transformationskompetenten rekombinanten H. saimiri.

Die Erfindung ist ferner in den Patentansprüchen und den Beispielen weiter ausgeführt.

Beispiel 1: Konstruktion von Plasmid pSIneo und pSIneoH

pSIneo und pSIneoH wurden so konstruiert, daß durch die homologe Rekombination mit H. saimiri DNA kein virales Gen deletiert und keine funktionelle Einheit unterbrochen wird.

Das "neo"-Gen kodiert für eine Phosphotransferase, die es ermöglicht, den durch Geneticin (G 418) induzierten Translationsblock in den meisten eukaryotischen Zellen zu umgehen.

Das Plasmid pSV2 neo (P.J. Southern und P. Berg (1982) J. Mol. Appl. Genetics, 327-341, Raven Press, New York) enthält das neo-Gen unter der Transkriptionskontrolle von SV40-Elementen (Früher Promoter/Enhancer, T-Antigen, mRNA Splice-Signal und Polyadenylierungsstellen). Die Transkriptionseinheit "neo" wurde aus pSV2 neo herausgeschnitten und die Restriktionsschnittstellen-Enden nach Standardmethoden von PvuII- in ClaI- bzw. von ECORI- in SalI-Enden umgewandelt. Die Klonierung des KpnI/SmaI Fragments E, das bei etwa 9 kb Länge das rechte Ende der L-DNA ausmacht, ist von KNUST (E. Knust et al. (1983) Gene 25, 281-289) beschrieben.

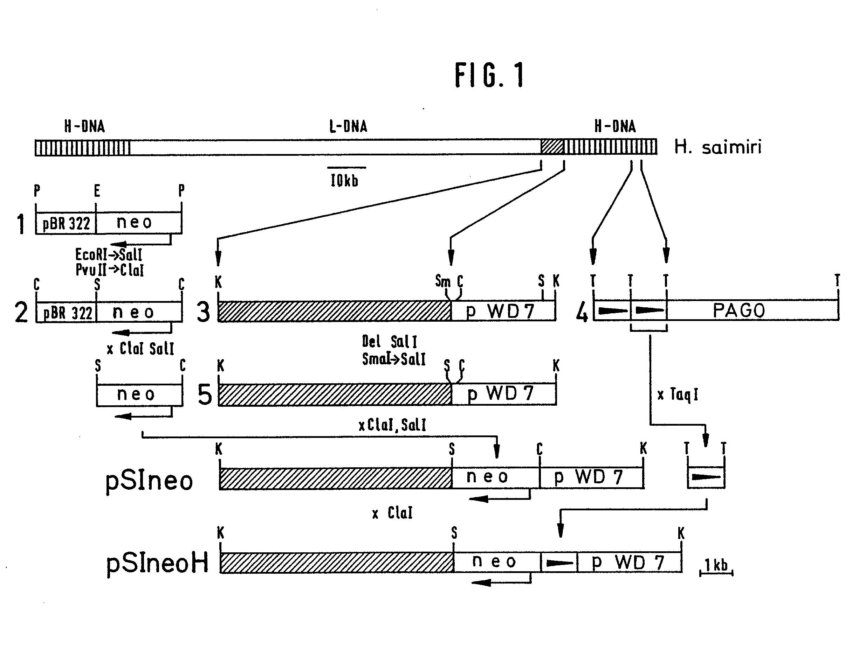

Der dort verwendete Klonierungsvektor pWD7 mit dem genannten Fragment E - als pWD11 (hier Vektor 3) bezeichnet -, wurde mittels Standardmethoden in den Vektor 5 umgewandelt, dadurch daß die interne SalI-Stelle in pWD7 deletiert und die SmaI-Stelle an der Fragment E/pWD7 Verbindung in eine SalI-Stelle mutiert ist. Die SmaI-bzw. SalI-Schnittstelle ist 35 Nukleotide vom Ende der L-DNA in der ersten H-Repetitionseinheit entfernt. Nach Schneiden von (5) mit SalI und ClaI kann nun das neo-Gen zwischen Fragment E und pWD7 in Vektor (5) zum Plasmid pSIneo einligiert werden. Eine H-DNA Repetitionseinheit von 1444 Basenpaaren (bp) wird schließlich nach Spaltung mit TaqI zwischen neo und pWD7 Anteil gesetzt, so daß schließlich Plasmid pSIneoH resultiert. Die H-DNA Repetitionseinheit wurde als TaqI-Fragment aus pFG24 erhalten (A.T. Bankier et al. (1985) J. Virol. 55, 133-139). Vorstehend genannte Syntheseschritte sind in Fig. 1 zusammengefaßt.

Beispiel 2: Kotransfektion von linearisierter pSIneoH-DNA mit H.saimiri Virion DNA (M-DNA)

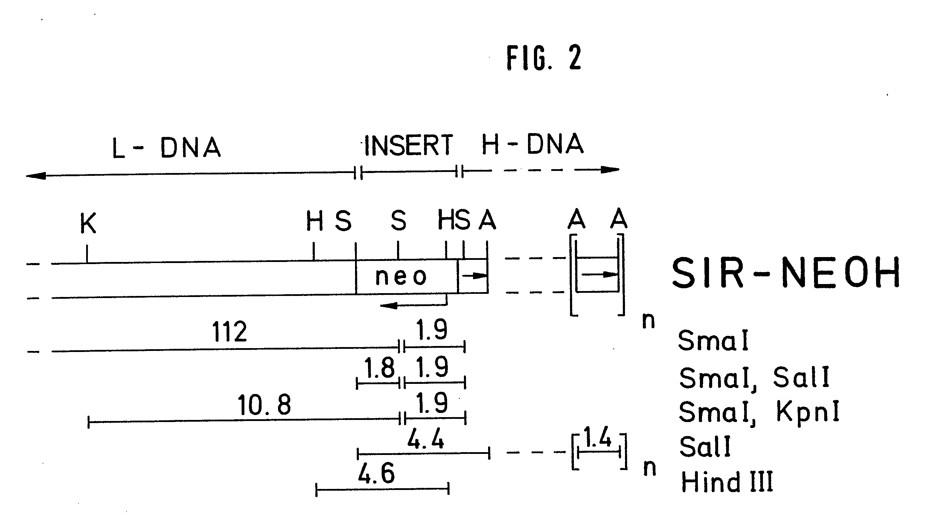

0,2 μg bis 0,4 μg über CsC1-Dichtegradienten gereinigte M-DNA (B. Fleckenstein et al. (1975) J. Virol. 15, 398-406) wurde mit 2 μg bis 4 μg durch KpnI linearisierter pSIneoH gemischt und in eine Owl Monkey Kidney Zelllinie (OMK - 637, ATCC CRL 1556) nach der Kalziumphosphat-Präzipitationsmethode transfiziert (F.L. Graham und J. van der Eg (1973) J. Virol. 52, 456-467), wobei ein 20% w/v Glycerin-Schock nach 4 Stunden erfolgte. Nach etwa 6 bis 10 Tagen waren erste zytopathische Effekte erkennbar und nach weiteren zwei Wochen waren die Zellen völlig lysiert. Durch die hohe Frequenz an spontaner Rekombination wurden rekombinante H.saimiri in der Regel ohne Selektionsdruck erhalten. Bei geringerer Anzahl von Rekombinanten wird mit Erfolg unter Selektionsdruck nach der Transfektion gearbeitet, wobei die Rekombinantenausbeute auf bis zu 80% steigt. Restriktionsanalyse mit SalI und SmaI in Verbindung mit Southern Blots zeigte, daß etwa zwei Drittel der Virus-Plaques ein vollständiges neo-Gen enthalten. Sechs von 9 Klonen hatten das neo-Gen bzw. pSIneoH DNA zwischen die H-DNA Repetitionseinheiten insertiert, zwei Klone hatten die neo-Sequenz in der Verbindungsstelle H/L insertiert. Einer dieser Klone, H.saimiri SIRneoH14 ist in Fig. 2 in Form seiner Genkarte charakterisiert. Alle H. saimiri SIRneoH Rekombinanten exprimieren das neo-Gen und sind in geeigneten Zellen, z.B. Sg 021, selektierbar, nachdem sie mit Virus aus den o.g. Kotransfektionen infiziert wurden. Sg 021-Zellen sind eine IL-2 abhängige T-Zellinie aus Marmosets, die mit H. saimiri persistent infizierbar ist (R.C. Desrosiers et al., (1985) Mol. Cell. Biol. 5, 2796-2803). Die Gendosis des neo-Gens steigt dabei mit zunehmender G 418 Konzentration von etwa 40 bei 100 μg/ml G 418 auf etwa 140 bei 750 μg/ml G 418.

Durch Kokultivation mit OMK-Zellen wird wiederum rekombinantes "Neo-Virus" aus den Sg 021-Zellen erhalten.

Beispiel 3: Kotransfektion von pSIneo DNA mit H. saimiri Virion DNA

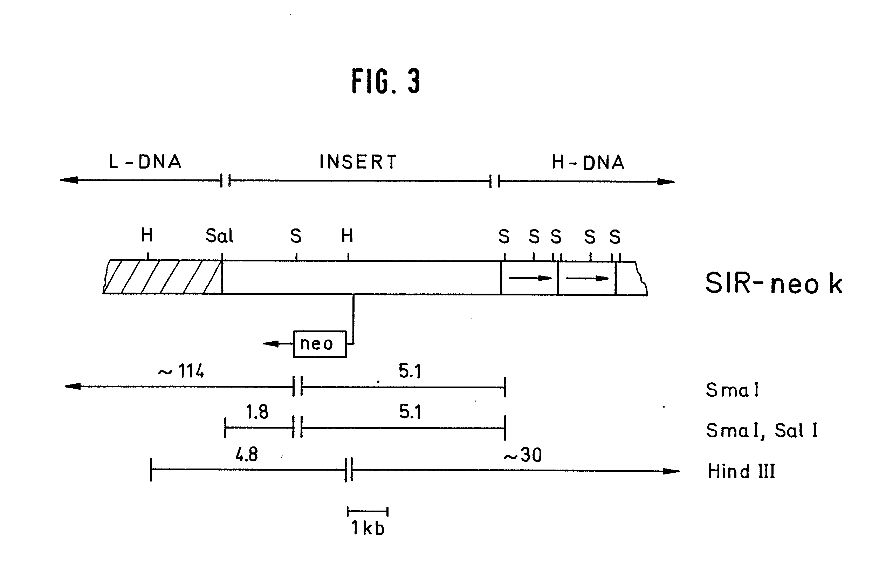

Um die Ausbeute an Rekombinanten zu erhöhen, die das neo-Gen in die H/L-Verbindungsstelle insertieren, wurde im Prinzip wie in Beispiel 2 kotransfiziert, jedoch hier mit linearisierter pSIneo DNA. Die aus dieser Transfektion resultierenden Viren H. saimiri SIRneoK hatten die neo-Sequenz innerhalb der pSIneo DNA zwischen die L/H Übergangsstelle insertiert, wie in Fig. 3 gezeigt. Expression und Amplifikation des neo-Gens sowie Produktion von infektiösem rekombinantem Virus erfolgte wie in Beispiel 2 beschrieben.

Beispiel 4: Persistente Infektion humaner T-Zellen mit rekombinantem H. saimiri Virus

Mit H. saimiri SIRneoH14 wurde die permanent wachsende humane T-Zellinie JURKAT (U. Schneider et al. (1977) Int. J. Cancer 19, 626; ATCC CRL 8163) infiziert und mit steigenden Konzentrationen Geneticin in RPMI 1640 Medium enthaltend 10% fötales Kälberserum kultiviert. Das neo-Gen war in den persistent infizierten JURKAT-Zellen in einer Gendosis von etwa 100 pro Zelle enthalten, was der Anzahl der H. saimiri SIRneoH14 Genome pro Zelle entspricht. Die virale DNA ist nicht in das zelluläre Genom integriert, sondern liegt als episomale DNA vor. Es waren in etwa 100 Kopien des rekombinanten Virus pro Zelle vorhanden.

Legende zu Figur 1:

1 bezeichnet das Plasmid PSV2 neo
2 bezeichnet das Plasmid PSV2 neo CS
3 bezeichnet das Plasmid pWD 11
4 bezeichnet das Plasmid pFG 24
C steht für ClaI; E für EcoRI; K für KpnI
S steht für SalI; Sm für SmaI
T steht für TaqI.

Die schraffierten Regionen bezeichnen den H. saimiri DNA-Anteil (terminales KpnI-SmaI Fragment der L-DNA), schwarze Dreiecke die H-DNA "Repeat"-Einheiten.

Legende zu Figur 2:

Der rechte H-/L-DNA Übergang von SIR-NEOH 14 ist dargestellt.
H steht für HindIII; K für KpnI; S für Sma I; Sa für SalI; A für die Spaltstelle der viralen Konkatemere. Die Zahlen bedeuten die Länge der DNA in kb.

Legende zu Figur 3:

siehe Figur 2

**Ansprüche**

1. H. saimiri Vektoren, dadurch gekennzeichnet, daß ein oder mehrere Fremdgene in der rechten oder linken Verbindungsregion der L- und H-DNA insertiert ist.

2. H. saimiri Vektoren nach Anspruch 1, dadurch gekennzeichnet, daß ein Selektionsgen in der rechten oder linken Verbindungsregion der L- und H- DNA insertiert ist.

3. Vektoren nach Anspruch 2, dadurch gekennzeichnet, daß das neo-Gen insertiert ist.

4. Vektoren nach Anspruch 2 oder 3, dadurch gekennzeichnet, daß nicht-onkogene H. saimiri-Varianten gewählt werden.

5. Verfahren zur Herstellung der Vektoren nach Anspruch 1, 2, 3 oder 4, dadurch gekennzeichnet, daß ein Selektionsgen und/oder ein oder mehrere Fremdgene in die rechte oder linke Verbindungsregion der L- und H-DNA einligiert wird.

6. Verfahren zur Herstellung der Vektoren nach Anspruch 1, 2, 3 oder 4, dadurch gekennzeichnet, daß die Ligation über Rekombination erfolgt.

7. Verwendung der Vektoren nach Anspruch 1, 2, 3 oder 4 zur Expression von Fremdgenen in humanen T-Zellen.

8. Verwendung der Vektoren nach Anspruch 1, 2, 3 oder 4 zur Expression von Fremdgenen in durch diese transformierten Affen-T-Lymphozyten.

9. Verfahren zur Expression von Fremdgenen in humanen oder Affen-T-Zellen, dadurch gekennzeichnet, daß solche Zellen durch Vektoren nach Patentansprüche 1 bis 8 transfiziert werden

1. Verfahren zur Herstellung von H. saimiri Vektoren, dadurch gekennzeichnet, daß ein oder mehrere Fremdgene in die rechte oder linke Verbindungsregion der L- und H-DNA einligiert werden.

2. Verfahren zur Herstellung von H. saimiri Vektoren, dadurch gekennzeichnet, daß ein Selektionsgen und/oder ein oder mehrere Fremdgene in die rechte oder linke Verbindungsregion der L- und H-DNA einligiert werden.

3. Verfahren nach Anspruch 1 oder Anspruch 2, dadurch gekennzeichnet, daß als Selektionsgen das neo-Gen einligiert wird.

4 Verfahren zur Herstellung von H. saimiri Vektoren nach Anspruch 1, 2 oder 3, dadurch gekennzeichnet, daß die Ligation uber Rekombinanten erfolgt.

5. Verfahren zur Expression von Fremdgenen in humanen oder Affen-T-Zellen, dadurch gekennzeichnet, daß soiche Zellen mit nach Anspruch 1, 2, 3 oder 4 hergestellten Vektoren transformiert werden.

# FIG. 1

EP 0 362 732 A2

FIG. 2

SIR-NEOH

EP 0 362 732 A2

FIG. 3

EP 0 362 732 A2